# EUROPEAN PATENT APPLICATION

(11) **EP 4 464 293 A1**
(43) Date of publication of application: **20.11.2024**
(21) Application number: 23796037.2
(22) Date of filing: 06.04.2023
(51) Int. Cl.: A61F 13/494, A61F 13/496

(54) **UNDERPANTS-STYLE DISPOSABLE WEARABLE ARTICLE**

(30) Priority: 27.04.2022 JP 2022073755
(71) Applicant: Daio Paper Corporation, Ehime 799-0492 (JP)
(72) Inventor: NAKAMARU, Hikari, Shikokuchuo-shi, Ehime 799-0113 (JP); FUJIWARA, Yuto, Shikokuchuo-shi, Ehime 799-0113 (JP)
(74) Representative: Williams Powell
(86) International application number: PCT/JP2023/014232
(87) International publication number: WO 2023/210291

(57) **Abstract**

Provided is a disposable wearing article in which texture deterioration of side non-stretchable regions of single planar gathers is reduced. In the article, each side flap has in its front and back end regions side non-stretchable regions each having a top part-under part fixed part. Each fixed part is spaced apart from the side edge of the non-stretchable region, between the side edge and the fixed part is a hollow area over its width, and the non-stretchable region has a free zone extending from its side edge. In a worn state of the article, the under part between the free zone and the fixed part is contracted with the stretchable lower torso region, which causes the under part of the free zone to stand up, while the top part of the free zone rises and billows, and part of the billowing hangs over the fixed part.

## Description

### FIELD OF ART

The present invention relates to an underpants-type disposable wearing article having planar gathers arranged on lateral sides opposed in the width direction of an inner member.

### BACKGROUND ART

Underpants-type disposable wearing articles, such as underpants-type disposable diapers and underpants-type sanitary products, usually include an outer member having a lower torso section of a front body section, a lower torso section of a back body section, and side seals formed by joining these two sections along their lateral sides opposed in the width direction; an inner member joined to the middle of the outer member in the width direction to extend from the lower torso section of the front body section, via the crotch section, up to the lower torso section of the back body section; a waist opening defined by the front edge of the front body section and the back edge of the back body section; and a pair of leg openings each formed on either side in the width direction of the inner member.

The lower torso sections of the outer member are provided with elastic members to impart stretchability in the width direction. With such stretchability, the lower torso sections fit well on the body surface of a wearer.

Recently, underpants-type disposable wearing articles are popular which are provided with side gathers arranged on the lateral sides opposed in the width direction of the inner member to fit on the inner thighs of a wearer, for the purpose of improved fitting around the legs and for so-called leak protection (for example, see Patent Publications 1 to 3).

Side gathers of various structures have hitherto been proposed. For example, gathers that stand up on the top side from the lateral sides of the inner member are generally called three-dimensional gathers, and provide superior leak protection. On the other hand, gathers that are formed by side flaps extending laterally from the absorber body (without being folded onto the top side) are generally called planar gathers, and are often used in combination with the three-dimensional gathers.

There have also been proposed particularly simple and low-cost products having planar gathers, but not three-dimensional gathers (see, for example, Patent Publications 1 to 4). Specifically, the side flaps disclosed in Patent Publication 4 each has a side nonwoven fabric which has a top part extending from the side edge of the absorber body to the side edge of the side flap and an under part folded along the side edge of the top part and extending to the side edge of the absorber body, and elongate gathering elastic members extending along the front-back direction between the top part and the under part. Each side flap has in its front and back end regions side non-stretchable regions each of which is not contracted in the front-back direction together with the gathering elastic members, and has a joined sheet area wherein the top and under parts are joined. The region of each side flap between the front and back end regions forms planar gathers, in which the gathering elastic members are fixed to the top and under parts, and which is contracted but stretchable in the front-back direction together with the gathering elastic members.

Planar gathers extending to overlap the stretchable regions of the lower torso sections of the outer member will result in formation of an area contracted both in the front-back direction and in the width direction, which disadvantageously deteriorates the wearing comfort or appearance. Thus, each side flap preferably has side non-stretchable regions in its front and back end regions. Here, the joined sheet area in each side non-stretchable region is provided for keeping the side non-stretchable region from forming a hollow cylinder as a whole which undergoes irregular deformation to deteriorate the wearing comfort.

However, the side non-stretchable regions disclosed in Patent Publication 4 have hard joined sheet areas, which may disadvantageously be exposed on the surface and deteriorate the texture.

### PRIOR ART PUBLICATION

### PATENT PUBLICATION

Patent Publication 1: JP 5303689 B
Patent Publication 2: JP 5400982 B
Patent Publication 3: JP 6986097 B
Patent Publication 4: JP 2016-083122 A

### SUMMARY OF THE INVENTION

### PROBLEM TO BE SOVED BY THE INVENTION

It is therefore a primary object of the present invention to reduce degradation of texture of the side non-stretchable regions of single planar gathers.

### MEANS FOR SOLVING THE PROBLEM

The underpants-type disposable wearing articles which solve the problem are as follows:

### <First Aspect>

An underpants-type disposable wearing article, including:
an outer member having a lower torso section of a front body section, a lower torso section of a back body section, and side seals formed by joining the two sections along their lateral sides opposed in a width direction,
an inner member joined to a middle of the outer member in the width direction to extend from the lower torso section of the front body section, via a crotch section, to the lower torso regio of the back body section,
a waist opening defined by a front edge of the front body section and a back edge of the back body section, and
a pair of leg openings each formed on either lateral side of the inner member,
wherein the inner member includes an absorber body extending from forward of the crotch section to backward of the crotch section,
wherein the lower torso section of the front body section and the lower torso section of the back body section each has stretchable lower torso regions provided at least on lateral sides opposed in the width direction,
wherein the stretchable lower torso regions each contains lower torso elastic members, and is contracted but stretchable in the width direction WD together with the lower torso elastic members,
wherein the inner member has on its opposed lateral sides a pair of side flaps extending laterally in the width direction from the side edges of the absorber body, each side flap extending in the front-back direction from an area overlapping the stretchable lower torso region of the front body section to an area overlapping the stretchable lower torso region of the back body section,
wherein each side flap has a side nonwoven fabric extending from a top face across a side edge down to an under face of the side flap, and elongate gathering elastic members each extending along the front-back direction between a top part and an under part of the side nonwoven fabric,
wherein the top part and the under part each extends all over the front-back direction of the side flap,
wherein each side flap has in its front and back end regions side non-stretchable regions each of which is not contracted in the front-back direction together with the gathering elastic members, and has a fixed part wherein the top part is directly or indirectly fixed to the under part,
wherein a region of each side flap between the front and back side non-stretchable regions forms a planar gather region, to which the gathering elastic members are fixed and which is contracted but stretchable in the front-back direction together with the gathering elastic members, and
wherein the article is devoid of three-dimensional gathers that stand up on a top side,
wherein the fixed part in each side non-stretchable region is spaced apart from a side edge of the side non-stretchable region toward a center in the width direction by a first distance,
wherein between the side edge and the fixed part of the side non-stretchable region is a hollow area all over its dimension in the width direction,
wherein the side non-stretchable region has a free zone not fixed to the outer member and extending from the side edge of the side non-stretchable region toward the center in the width direction by a second distance,
wherein the second distance is larger than half a dimension in the width direction of the fixed part and smaller than half the first distance,
wherein the under part located between the free zone and the fixed part is joined to the stretchable lower torso region of the outer member, and is contracted but stretchable in the width direction together with the lower torso elastic members, and
wherein a maximum elongation in the width direction of each stretchable lower torso region is 200 to 320%.

### <Function and Effect>

According to the underpants-type disposable wearing article of the present invention, supposing that the maximum elongation in the width direction of each stretchable lower torso region is Emax, when each stretchable lower torso region is in a state of about 0.5 to 0.7 Emax (equivalent to the worn state), in each of the side non-stretchable regions, the under part of the zone between the free zone and the fixed part is contracted in the width direction together with the stretchable lower torso region, which causes the under part of the free zone to stand up, while the top part of the free zone rises and billows. The area of the top part adjacent to the fixed part (non-fixed area of the side nonwoven fabric) hangs over at least the side edge area of the surface of the fixed part. Further, the billowing is flattened and extended when the article is worn, to thereby cover the fixed part even more widely. As a result, the surface of the hard fixed part 67 in the side non-stretchable region 70 is hard to be brought into contact with the skin, which reduces deterioration of the texture.

### <Second Aspect>

The underpants-type disposable wearing article according to the first aspect,
wherein the first distance is 15 to 20 mm,
wherein the second distance is one third or less of the first distance, and
wherein a dimension in the width direction of the fixed part is 3 mm or more.

### <Function and Effect>

The dimensions of the various areas, zones, regions, or the like may suitably be decided, and are particularly preferably within the ranges of this aspect.

### <Third Aspect>

The underpants-type disposable wearing article according to the first or second aspect,
wherein, where the first distance is w1, the second distance is w2, and the dimension in the width direction of the fixed part is w3, a formula 0.3 (w1 + w2) / 2 ≥ w3 is satisfied.

<Function and Effect>

The dimensions of the various areas, zones, regions, or the like may suitably be decided, and are particularly preferably within the ranges of this aspect for more billowing of the top part to allow easy covering of the fixed parts widely.

### <Fourth Aspect>

The underpants-type disposable wearing article according to any one of first to third aspects,
wherein the second distance is larger than the dimension in the width direction of the fixed part.

### <Function and Effect>

The dimensions of the various areas, zones, regions, or the like may suitably be decided, and are particularly preferably within the ranges of this aspect for easier billowing of the top part.

### <Fifth Aspect>

The underpants-type disposable wearing article according to any one of first to fourth aspect,
wherein an area of the under part included in the fixed part is joined to the stretchable lower torso region of the outer member, and is contracted but stretchable in the width direction together with the lower torso elastic members.

### <Function and Effect>

According to this aspect, when the stretchable lower torso regions are in a state of about 0.5 to 0.7 Emax (equivalent to the worn state), the fixed parts are contracted in the width direction together with the stretchable lower torso regions to become smaller, so that the coverage of the fixed parts with the top parts are relatively larger. Thus, the surfaces of the fixed parts are more hardly brought into contact with the skin.

### <Sixth Aspect>

The underpants-type disposable wearing article according to any one of first to fifth aspects,
wherein the fixed part is spaced laterally apart from a side edge of the absorber body by a third distance,
wherein the top part has a terminal zone protruding from the fixed part centrally in the width direction by a fourth distance, and not joined to any member adjacent underside thereof,
wherein the fourth distance is larger than half a dimension in the width direction of the fixed part, and
wherein an area of the under part located between the fixed part and a side edge of the absorber body is joined to the stretchable lower torso region of the outer member, and is contracted but stretchable in the width direction together with the lower torso elastic members.

### <Function and Effect>

According to the present aspect, the terminal zone of the top part protruding from the fixed part centrally in the width direction rises along contraction creases of the member located underside thereof, and the risen area of the top part adjacent to the fixed part (non-fixed area of the side nonwoven fabric) hangs over at least the side edge area of the surface of the fixed part closer to the center in the width direction. As a result, the surface of the fixed part is more hardly brought into contact with the skin.

### EFFECT OF THE INVENTION

As discussed above, the present invention provides advantages, such as reduced deterioration of texture of the side non-stretchable regions of single planar gathers.

### BRIEF DESCRIPTION OF THE DRAWINGS

Fig. 1 is a plan view of an underpants-type disposable diaper in its spread state, showing its top face.
Fig. 2 is a plan view of the underpants-type disposable diaper in its spread state, showing its under face.
Fig. 3 is a cross-sectional view taken along lines 3-3 in Fig. 1.
Fig. 4 is a cross-sectional view taken along lines 4-4 in Fig. 1.
Fig. 5 is a sectional view taken along lines 5-5 in Fig. 1.
Fig. 6 is a plan view of the inner member, showing its top face.
Fig. 7 is a perspective view of the underpants-type disposable diaper seen from the lower front.
Fig. 8 is a sectional view of a relevant part of the diaper in a worn state.
Fig. 9 is a cross-sectional view of the absorbent element.
Fig. 10 is a plan view of the absorbent element.
Fig. 11 is a plan view of a relevant part of Fig. 10.
Fig. 12 is an enlarged cross-sectional view of a relevant part of a side non-stretchable region in a spread state.
Fig. 13 is an enlarged cross-sectional view of the relevant part of the side non-stretchable region, contracted to 70% of the original size.
Fig 14 is a front view schematically illustrating inclination of various parts of the side flaps.

### MODES FOR CARRYING OUT THE INVENTION

The underpants-type disposable wearing articles mentioned above will now be discussed in detail with reference to an underpants-type disposable diaper as an example shown in the attached drawings. Note that components adjacent to each other in the thickness direction may be fixed or joined as necessary in the same way as in known diapers, at locations other than the fixed or fixed parts to be discussed below. Dotted pattern regions in the sectional views represent an adhesive, such as a hot melt adhesive, as fixing or joining means. The hot melt adhesive may be applied by any known manner, such as slot coating, continuous or intermittent linear bead coating, spray coating in spiral, Z-shaped, or wave (regular or irregular) patterns, or pattern coating (transfer of a hot melt adhesive by relief printing). In place of or in addition to this, on fixing portions of elastic members, a hot melt adhesive may be applied to the external surface of the elastic members for fixing to adjacent members. Examples of the hot melt adhesive include, but not limited to, EVA-based, adherent rubber-based (elastomer-based), polyolefin-based, and polyester/polyamide-based adhesives. The fixing or joining means for joining components may alternatively be material melt-bonding, such as heat sealing or ultrasonic sealing. Components adjacent to each other in the thickness direction are fixed or joined in an intermittent pattern, where liquid permeability in the thickness direction is required. For example, for such intermittent fixing or joining with a hot melt adhesive, intermittent pattern coating in a spiral, Z, or wave shape may preferably be employed. For application in an area wider than the application width of one nozzle, intermittent pattern coating in a spiral, Z, or wave shape may be employed with or without space in the width direction. The joining means for joining components may be material melt-bonding, such as heat sealing or ultrasonic sealing.

As the nonwoven fabric in the description hereinbelow, commonly known nonwoven fabric may suitably be used depending on the parts or purposes. Examples of the constituent fibers of the nonwoven fabric include, but not limited to, synthetic fibers, such as polyolefin-based, e.g., polyethylene or polypropylene, polyester-based, or polyamide-based fibers (including not only single component fibers, but also composite fibers, such as of core/sheath type), as well as regenerated fibers, such as rayon or cupra, or natural fibers, such as cotton, and also mixtures thereof. For improved flexibility of the nonwoven fabric, the constituent fibers may preferably be crimped fibers. The constituent fibers of the nonwoven fabric may also be hydrophilic fibers (including those rendered hydrophilic with hydrophilizers), hydrophobic fibers, or water-repelling fibers (including those rendered water-repelling with water repellents). Further, nonwoven fabric may generally be categorized into discontinuous fiber nonwoven, continuous fiber nonwoven, spunbonded nonwoven, melt blown nonwoven, spunlace nonwoven, thermal bonded (air through) nonwoven, needle-punched nonwoven, point-bonded nonwoven, composite nonwoven (including SMS or SMMS nonwoven fabric having one or more melt blown layers interposed between spunbonded layers), or the like nonwoven fabric, generally depending on the length of the fibers, method of forming the sheet, method of joining the fibers, or layered structure, and any of these nonwoven fabric may be used.

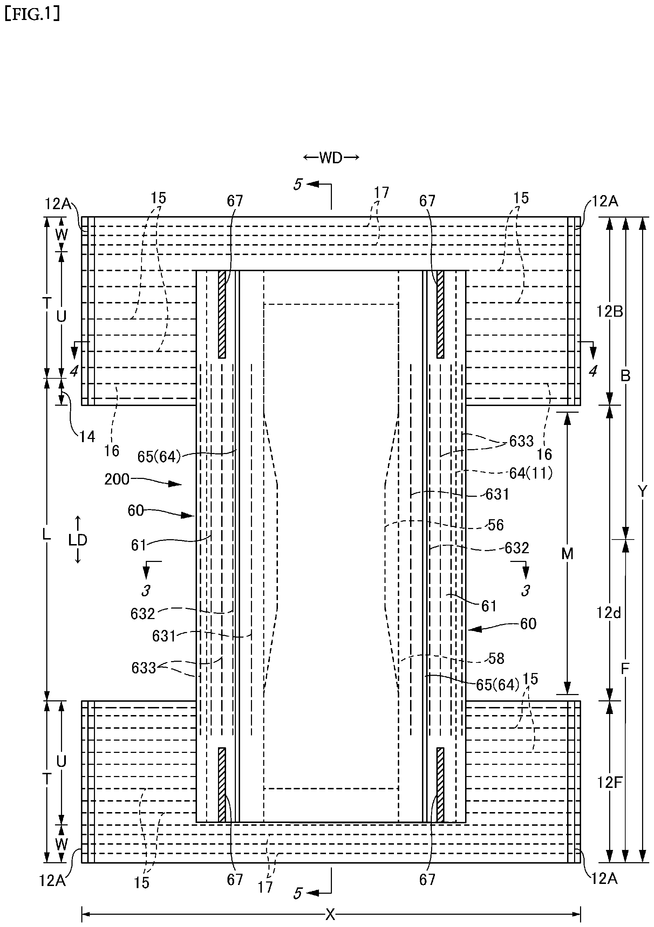

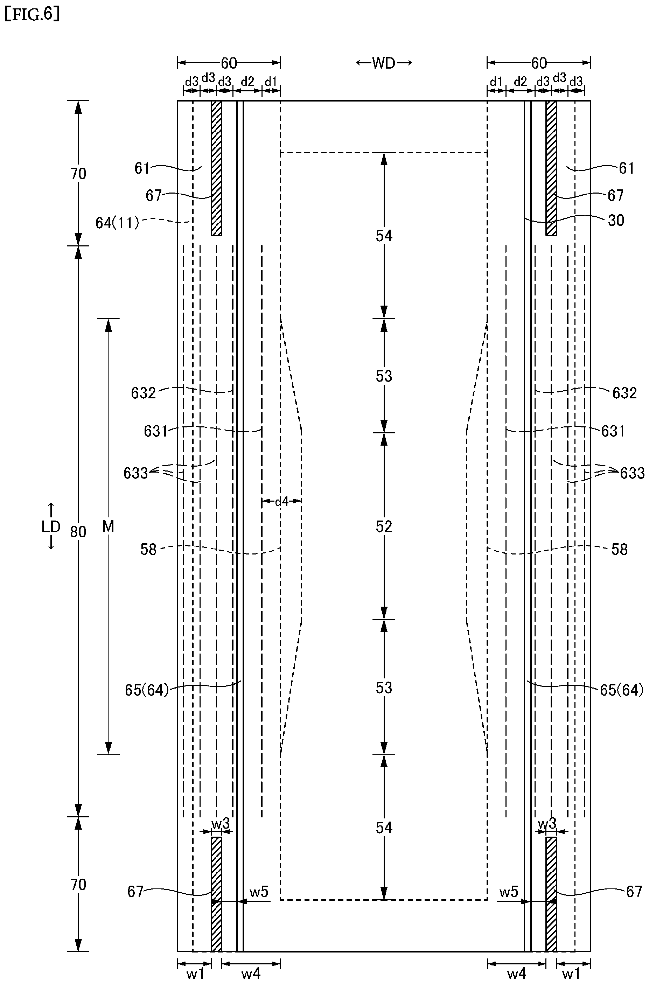

Figs. 1 to 7 show an embodiment of an underpants-type disposable diaper. The underpants-type disposable diaper 100 according to the invention has a rectangular front outer member 12F forming a front lower torso section, a rectangular back outer member 12B forming a back lower torso section, and an inner member 200 joined to the middle of each of the outer members 12F, 12B in the width direction to extend from the front outer member 12F, via the crotch section M, to the back outer member 12B. The front outer member 12F and the back outer member 12B are joined along their opposed lateral sides to form a pair of side seals 12A, to thereby define an opening with the front and back edges of the outer members 12F, 12B, which is a waist opening WO for passing the torso of the wearer therethrough, whereas a pair of leg openings LO is formed on either side in the width direction WD of the inner member 200, defined by the lower edges of the outer members 12F, 12B and the side edges of the inner member 200. The inner member 200 absorbs and holds bodily waste, such as urea, while the outer members 12F, 12B support the inner member 200 with respect to the body of a wearer. Reference sign Y refers to the entire length of the diaper in its spread state (the length in the front-back direction from the edge of the waist opening WO in the front body section over to the edge of the waist opening WO in the back body section), whereas reference sign X refers to the entire width of the diaper in its spread state.

The underpants-type disposable diaper 100 according to the present invention has lower torso regions T defined as front-back regions including the side seals 12A (front-back regions extending from the waist opening WO to the upper edge of each leg opening LO) and an intermediate section L defined as a front-back region forming the leg openings LO (a region between the front-back region of the front body section along the side seals 12A and the front-back region of the back body section along the side seals 12A). The regions located in the lower torso regions T of the front outer member 12F and the back outer member 12B, i.e., the front lower torso section and the back lower torso section may each be divided conceptually into a waist zone W forming the edge zone of the waist opening and an under-waist zone U extending downward of the waist zone W. Usually, when the front lower torso section and the back lower torso section each has one or more boundaries between zones of different stretching stresses in the width direction (e.g., with elastic members of different finenesses or stretch rates), the zone on the waist opening WO side of the boundary closest to the waist opening WO is the waist zone W. In the absence of such boundaries, the zones of the front and back lower torso sections extending toward the waist opening WO beyond the absorber body 56 or the inner member 200 are the waist zones W. The length in the front-back direction of the waist zones may vary depending on the size of the product, and may suitably be decided. For example, each waist zone W may extend over 15 to 40 mm and each under-waist zone U may extend over 65 to 120 mm. On the other hand, the side edges of the intermediate section L are centrally narrowed in square U-shapes or curved shapes to fit the round-leg profile of the wearer, and the legs of the wearer are to be inserted here. Thus, underpants-type disposable diapers in the spread state have generally an hourglass shape as a whole.

### <Outer Member>

The outer member may be composed of a rectangular front outer member 12F forming at least the lower torso section of the front body section F and a rectangular back outer member 12B forming at least the lower torso section of the back body section B, as in the illustrated embodiment, and the front outer member 12F and the back outer member 12B may be non-continuous on their crotch sides and spaced apart in the front-back direction LD (two-segmented outer member). Alternatively, though not shown, the front body section F and the back body section B may be continuous via the crotch section M (integral outer member). The spaced distance 12d in the front-back direction in the two-segmented outer member may be, for example, about 40 to 60% the overall length Y. In the illustrated embodiment, the lower edges of the front outer member 12F and the back outer member 12B extend linearly along the width direction WD, but at least one of the lower edges of the front and back outer members 12F and 12B may be curved along the round-leg profile.

The underpants-type disposable diaper with the two-segmented outer member, wherein the inner member 200 is exposed between the front outer member 12F and the back outer member 12B, is preferably provided, on the under face of the inner member 200, with a covering nonwoven layer 13 extending from between the front outer member 12F and the inner member 200 to between the back outer member 12B and the inner member 200 so that a liquid impervious film 11 is not exposed on the under face of the inner member 200. The top and under faces of the covering nonwoven layer 13 may be adhered to the surfaces facing these faces, respectively, via a hot melt adhesive. The covering nonwoven layer 13 may be made of, for example, nonwoven fabric suitably selected from those forming the outer members 12F, 12B.

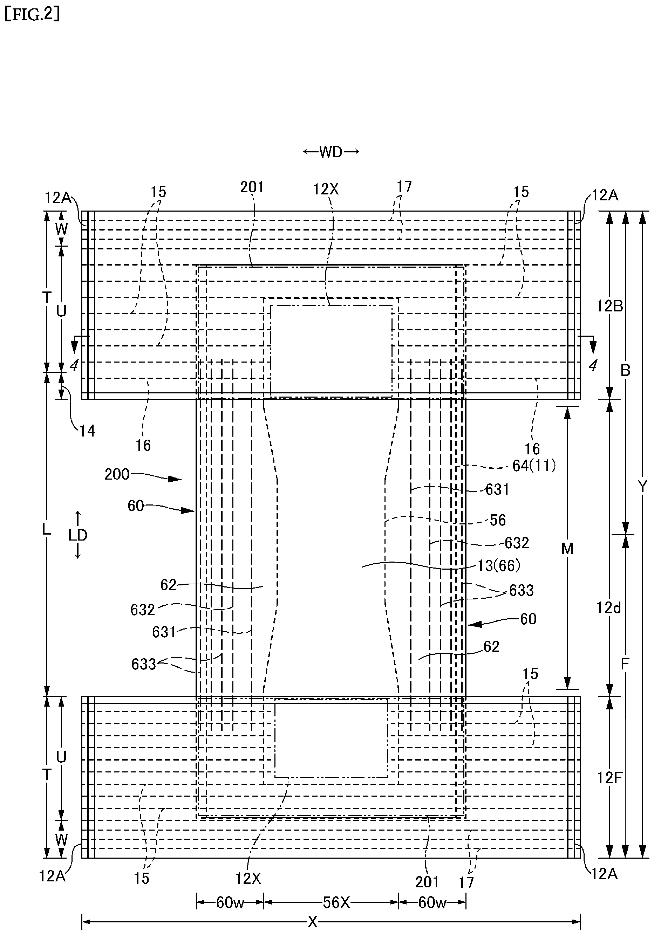

The front outer member 12F and the back outer member 12B have the front lower torso section and the back lower torso section, respectively, forming the lower torso regions T. In the embodiment shown in Figs. 1 and 2, the back outer member 12B has a larger dimension in the front-back direction compared to the front outer member 12F, the front outer member 12F has no zone corresponding to the intermediate region L, and the back outer member 12B has a buttocks-covering zone 14 extending from the lower torso region T toward the intermediate region L. However, the front outer member 12F and the back outer member 12B may have the same dimension in the front-back direction LD, and neither the front outer member 12F or the back outer member 12B may have any zone corresponding to the intermediate region L.

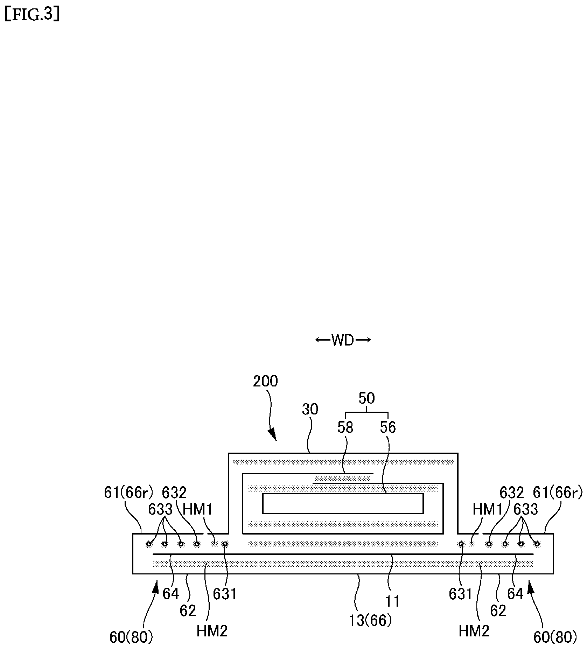

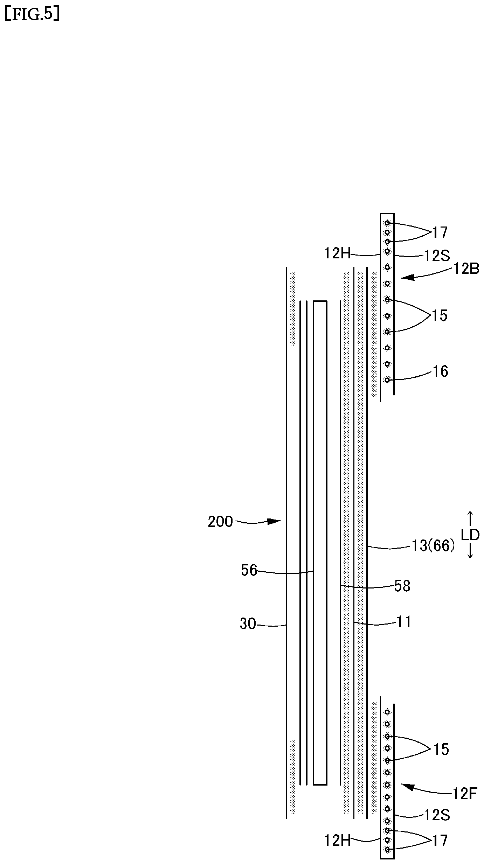

Each outer member 12F, 12B is formed by joining an outer sheet layer 12S and an inner sheet layer 12H adjacent outward and inward, respectively, to elastic members 15 to 17 to be discussed later, with joining means, such as a hot melt adhesive or welding, as shown in Figs. 3 to 5. The outer sheet layer 12S and the inner sheet layer 12H may be formed by folding a single sheet material to position the folding line on the waist opening side, as shown in Fig. 5, or, though not shown, by adhering two sheet materials together.

The sheet material used for the outer sheet layer 12S and the inner sheet layer 12H may be any material without particular limitation, and may preferably be nonwoven fabric. The nonwoven fabric, where used, preferably has a basis weight of about 10 to 30 g/m² per sheet.

For improved fitting on the lower torso of the wearer, each outer member 12F, 12B is provided with elastic members 15 to 17 attached at a predetermined stretch rate between the outer sheet layer 12S and the inner sheet layer 12H to form stretchable lower torso regions which are elastically stretchable with the elastic members 15 to 17 in the width direction WD. Each stretchable lower torso region in the natural length state is contracted with the contraction of the elastic members 15 to 17 to form creases or ridges therein, whereas each stretchable lower torso region is stretchable in the longitudinal direction of the elastic members 15 to 17 up to the predetermined stretch rate where the stretchable lower torso region is fully stretched without creases. The elastic members 15 to 17 may be elongate elastic members, such as rubber threads (as illustrated in the figures), or any conventionally known elastic members, such as of a tape, net, or film shape, without particular limitation. The elastic members 15 to 17 may either be made of synthetic rubber or natural rubber. Bonding between the outer sheet layer 12S and the inner sheet layer 12H in each outer member 12F, 12B, or fixing of the elongate elastic members 15 to 17 therebetween, may be carried out with at least one of the fixing means including a hot melt adhesive applied in a various manner and material melt-bonding, such as heat sealing or ultrasonic sealing. It is preferred not to bond or to merely weakly bond the elongate elastic members 15 to 17 in their areas other than the bonding areas, since strongly fixing the elastic members all over the outer members 12F, 12B will result in impaired flexibility of the outer members. In the illustrated embodiment, by holding between the sheet layers 12S and 12H the elongate elastic members 15 to 17, only to the circumferential surfaces of which a hot melt adhesive is applied using application means, such as comb guns or surewrap nozzles, not only the fixing of the elongate elastic members 15 to 17 to the sheet layers 12S, 12H, but also the fixing between the sheet layers 12S and 12H, may be achieved by means of only the hot melt adhesive applied on the circumferential surfaces of the elongate elastic members 15 to 17.

The elastic members 15 to 17 in the illustrated embodiment will now be discussed in further detail. Between the outer sheet layer 12S and the inner sheet layer 12H in the waist zone W of each outer member 12F, 12B, a plurality of waist elastic members 17 is attached at intervals in the front-back direction, with each member 17 being stretched in the width direction at a specific stretch rate, to thereby form a waist stretchable zone continuously extending all over the width direction WD. One or a plurality of the waist elastic members 17 arranged adjacent to the under-waist zone U may be arranged coincident with the inner member 200, or may be arranged on opposed lateral sides of the inner member 200 in the width direction WD, except for a middle region, which is overlapped with the inner member 200. As the waist elastic members 17, about two to fifteen, particularly about four to ten rubber threads, each having a fineness of 155 to 1880 dtex, particularly about 470 to 1240 dtex (in the case of synthetic rubber) (in the case of natural rubber, with a cross-sectional area of 0.05 to 1.5 mm², particularly about 0.1 to 1.0 mm²), are preferably arranged at 2 to 12 mm intervals, particularly 3 to 7 mm intervals, to render the waist zone W to have a stretch rate in the width direction WD of preferably 150 to 400%, particularly about 220 to 320%. Further, the waist zone W may not necessarily contain the elastic members of the same fineness or the same stretch rate all over the front-back direction LD, and may contain elastic members having partially different finenesses or stretch rates.

Between the outer sheet layer 12S and the inner sheet layer 12H in the under-waist zone U of each outer member 12F, 12B, a plurality of under-waist elastic members 15, which is a plurality of elongate elastic members, is attached at intervals in the front-back direction, with each member 15 being stretched in the width direction at a specific stretch rate, to thereby form under-waist stretchable regions arranged on opposed lateral sides of the inner member 200 in the width direction WD, except for a middle region, which is overlapped with the inner member 200. As the under-waist elastic members 15, about five to thirty rubber threads, each having a fineness of 155 to 1880 dtex, particularly about 470 to 1240 dtex (in the case of synthetic rubber) (in the case of natural rubber, with a cross-sectional area of 0.05 to 1.5 mm², particularly about 0.1 to 1.0 mm²), are preferably arranged at 1 to 15 mm intervals, particularly 3 to 8 mm intervals, to render the under-waist zone U to have a stretch rate in the width direction WD of preferably 200 to 350%, particularly about 240 to 300%. Further, the under-waist zone U may not necessarily contain the elastic members of the same fineness or the same stretch rate all over the front-back direction LD, and may contain elastic members having partially different finenesses or stretch rates.

Between the outer sheet layer 12S and the inner sheet layer 12H in the buttocks-covering zone 14 of the back outer member 12B, one or a plurality of buttocks-covering elastic members 16, which is one or a plurality of elongate elastic members, is attached at intervals in the front-back direction, with each member 16 being stretched in the width direction at a specific stretch rate, to thereby form buttocks-covering stretchable zones arranged on opposed lateral sides of the inner member 200 in the width direction WD, except in a middle region, which is overlapped with the inner member 200. As the buttocks-covering elastic members 16, about two to ten rubber threads, each having a fineness of 155 to 1880 dtex, particularly about 470 to 1240 dtex (in the case of synthetic rubber) (in the case of natural rubber, with a cross-sectional area of 0.05 to 1.5 mm², particularly about 0.1 to 1.0 mm²), are preferably arranged at 5 to 40 mm, particularly 5 to 200 mm intervals and fixed at a stretch rate of 150 to 300%, particularly 180 to 260%.

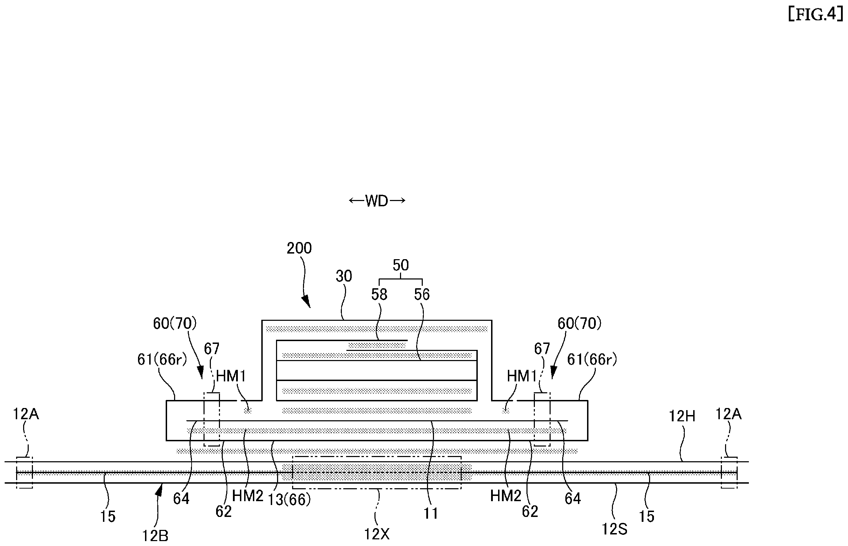

As in the illustrated embodiment, with the under-waist elastic members 15 and the buttocks-covering elastic members 16 being arranged, except in the middle areas, which are overlapped with the inner member 200, on opposed lateral sides of the inner member 200 in the width direction WD, the inner member 200 will not be contracted in the width direction more than necessary, and will not have bulky, inferior appearance or inferior absorption performance. Such a structure not only encompasses an embodiment wherein the elastic members 15, 16 are present only on the opposite lateral sides in the width direction WD, but also an embodiment wherein the elastic members 15, 16 are present extending from one lateral side across the inner member 200 to the other lateral side in the width direction, but are finely cut, as indicated with reference sign 12X in Figs. 2 and 4, partially in or all over the middle area, which is overlapped with the inner member 200, so that the contraction force will not act in that area (this is substantially equivalent to the absence of the elastic members 15, 16), so that contractible areas wherein the contraction force acts are provided only on the lateral sides opposed in the width direction WD. Naturally, the arrangements of the under-waist elastic members 15 and the buttocks-covering elastic members 16 are not limited to the embodiments discussed above, and part or all of the under-waist elastic members 15 and the buttocks-covering elastic members 16 may extend from one lateral side across the inner member 200 to the other lateral side in the width direction so that the contraction force will act all over the width direction, including the area overlapped with the inner member 200.

<Inner Member Joint Area>

The inner member 200 may be fixed to the outer members 12F, 12B by joining means, such as material melt-bonding, including heat sealing or ultrasonic sealing, or by a hot melt adhesive. In the illustrated embodiment, the under face of the inner member 200 is fixed to the top faces of the outer members 12F and 12B via a hot melt adhesive HM3. This inner member joint area 201 may be provided almost all over the overlapping area between the inner member and each outer member 12F, 12B, for example, in the region except for opposed lateral sides in the width direction of the inner member 200.

### <Inner Member>

The inner member 200 may be in any shape and, in the illustrated embodiment, is in a rectangular shape. The inner member 200 has a liquid-pervious top sheet 30 to be disposed on the side of the skin of the wearer, a liquid impervious film 11, and an absorbent element 50 interposed therebetween, as shown in Figs. 3 to 5. Reference numeral 60 refers to a side flap 60 having planar gathers.

### <Top Sheet>

The top sheet 30 is not particularly limited as long as it is made of a liquid-pervious material, such as perforated or non-perforated nonwoven fabric or porous plastic sheet. As in the embodiment illustrated in Figs. 3 and 4, the top sheet 30, when also acts as covering of a liquid-impervious film layer 64, may preferably be made of nonwoven fabric.

When the opposed lateral sides in the width direction WD of the top sheet 30 do not act as the covering of the liquid-impervious film layer 64, each lateral side of the top sheet 30 may be folded along the side edge of the absorbent element 50 to extend toward the underside thereof between the absorbent element 50 and the liquid-impervious film 11.

### intermediate Sheet>

Though not shown in the drawings, an intermediate sheet (also termed as a second sheet) may be provided, which has a higher liquid transmission rate compared to the top sheet 30, so as to have the liquid permeated through the top sheet 30 promptly transferred to the absorber body. This intermediate sheet promptly transfers liquid to the absorber body to improve the absorption performance of the absorber body, while it keeps the absorbed liquid from "back flowing" from the absorber body. The intermediate sheet may be omitted.

### <Liquid-Impervious Film>

The liquid-impervious film 11 provided on the underside of the absorber body 56 may be made of any material without particular limitation, for example, plastic film made of a polyolefin-based resin, such as polyethylene or polypropylene, or the like. The liquid-impervious film 11 is preferably made of a material having both liquid-impermeability and moisture-permeability, which material is preferably used recently for preventing dampness. Such moisture-permeable plastic film may be microporous plastic film, which is widely used and obtained by kneading an inorganic filler in a polyolefin-based resin, such as polyethylene or polypropylene, in a molten state, forming the resulting mixture into a sheet, and then uni- or biaxially drawing the sheet.

The liquid-impervious film 11 may extend, as in the embodiment illustrated in Figs. 3 and 4, into the side flaps to provide the planar gathers with improved waterproof property, or may have a width to fit underside the absorbent element 50, or may be folded along the side edges of the absorbent element 50 back toward top side to extend to between the absorbent element 50 and the top sheet 30.

### <Absorbent Element>

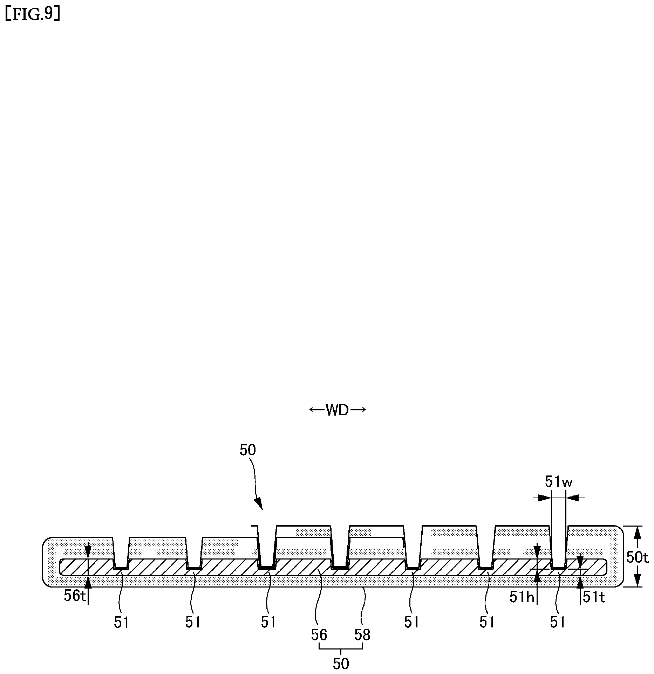

The absorbent element 50 includes, as shown in Figs. 3, 4, and 9, the absorber body 56 and a packing sheet 58 that entirely wraps the absorber body 56. The packing sheet 58 may be omitted.

### <Absorber Body>

The absorber body 56 may be formed of an assembly of fibers. Such an assembly of fibers may be an accumulation of short fibers of fluff pulp, synthetic fibers, or the like, as well as an assembly of filaments obtained by opening, where necessary, a tow (fiber bundle) of synthetic fibers, such as cellulose acetate. The basis weight of the fibers may be about 100 to 300 g/m² for an accumulation of fluff pulp or short fibers, and about 30 to 120 g/m² for an assembly of filaments. The fineness of the synthetic fibers, when used, is, for example, 1 to 16 dtex, preferably 1 to 10 dtex, more preferably 1 to 5 dtex.

The assembly of fibers may preferably have superabsorbent polymer particles mixed therein. The superabsorbent polymer particles include not only "particles", but also "powders". The superabsorbent polymer particles may be those used in this type of disposable diapers as they are, and may preferably be particles 30 wt% or less of which, after sieving (five-minute shaking), remain on a 500 µm standard sieve (JIS Z8801-1: 2006) and particles 60 wt% or more of which, after sieving (five-minute shaking), remain on a 180 µm standard sieve (JIS Z8801-1: 2006).

Any material of the superabsorbent polymer particles may be used without particular limitation, and those having a water absorption (according to JIS K 7223: 1996 "Testing Method for Water Absorption Capacity of Super Absorbent Polymers") of 40 g/g or more are preferred. The superabsorbent polymer particles may be formed of starch-based, cellulose-based, or synthetic polymer-based. Starch-acrylic acid (salt) graft copolymers, saponified products of starch-acrylonitrile copolymers, cross-linked sodium carboxymethyl cellulose, or acrylic acid (salt) polymers may be used. The superabsorbent polymer particles may preferably be in ordinary powder or granular form, but particles in other forms may also be used.

The superabsorbent polymer particles having a water absorption rate of 70 seconds or less, particularly 40 seconds or less, may preferably be used. With too slow a water absorption rate, so-called back flow may likely to occur, wherein liquid supplied into the absorber body 56 returns out of the absorber body 56.

The superabsorbent polymer particles may preferably be those having a gel strength of 1000 Pa or higher. With such property, when the superabsorbent polymer particles are formed into a bulky absorber body 56, stickiness after liquid absorption may effectively be limited.

The basis weight of the superabsorbent polymer particles may suitably be decided depending on the absorption amount required in a use of the absorber body 56. Thus, it depends, but the basis weight may be 50 to 350 g/m². At a basis weight of the polymer less than 50 g/m², the absorption amount may hardly be secured. At over 350 g/m², the effect may be saturated.

The ratio of the fibers to the superabsorbent polymer particles in the absorber body 56 is not particularly limited, and may be 50 : 50 to 20 : 80 by weight for a thinner absorber body 56 with the same area and the same amount of absorption. Here, the thickness 56t of the absorber body 56 is not particularly limited, and may be 3 to 15 mm.

The absorber body 56 extends continuously from forward of the crotch section M to backward of the crotch section M, and may preferably extend from the lower torso section W, U of the front body section F to the lower torso section W, U of the back body section B, as in the illustrated embodiment. Reference sign 56X refers to the maximum width (entire width) of the absorber body 56.

For ensuring the amount of absorption in the crotch section M, the absorber body 56 may preferably be in a generally rectangular shape, but when the absorber body 56 has a narrowest zone 52 in the crotch section M and enlarging zones 53 extending from the narrowest zone 52 toward maximum width zones 54 forward or backward of the crotch section M, as shown in Figs. 1, 2, 10 and 12, the narrowed zones 52, 53 composed of the narrowest zone 52 and the enlarging zones 53 preferably impart improved fitting on the inner thighs. In the latter embodiment, for ensuring the amount of absorption in the crotch section M, it is preferred that the narrowest width 52w of the absorber body 56 in the crotch section M is 0.8 times or more the maximum width 56X of the absorber body 56. The front-back dimension of the narrowed zones 52, 53 is preferably about 20 to 30% the entire length Y of the product.

Note that the crotch section M, when the absorber body 56 has the narrowed zones 52, 53 as discussed above, means the extent in the front-back direction LD including the narrowed zones 52, 53, whereas the crotch section M, when the absorber body 56 does not have the narrowed zones 52, 54 but the contour of the wearing article in the spread state has a narrowed zone as in the illustrated embodiment, means the extent in the front-back direction LD including this narrowed zone of the contour (in the illustrated embodiment, between the front outer member 12F and the back outer member 12B), or where the absorber body 56 has neither of the narrowed zones, the crotch section M means the extent in the front-back direction LD corresponding to 20 to 30% the entire length Y of the product and located in the middle in the front-back direction LD.

### <Packing Sheet>

The material of the packing sheet, when used, may be tissues, in particular, crepe paper, nonwoven fabric, polyethylene-laminated nonwoven fabric, perforated sheet, or the like, provided that preferably the superabsorbent polymer particles would not fall out of the sheet. When nonwoven fabric is used in place of the crepe paper, hydrophilic SMS nonwoven fabric (SMS, SSMMS, or the like) is particularly preferred, and those made of polypropylene, a polyethylene/polypropylene composite material, or the like may be used. The basis weight of packing sheet is preferably 5 to 40 g/m², particularly 10 to 30 g/m².

How to pack with the packing sheet 58 may suitably be decided and, in view of readiness of production or protection against leakage of the superabsorbent polymer particles through the front or back end edge, preferably the packing sheet 58 is wrapped cylindrically around the absorber body 56 to surround its top and under faces as well as both side faces, with the front and back edge portions of the packing sheet extending forwardly and backwardly beyond the absorber body 56, and the front and back extensions are pressed in the top-under direction and joined with joining means, such as a hot melt adhesive or material melt-bonding.

### <Side Flaps>

As shown in Figs. 1 to 4, 6, and 12, side flaps 60 refer to a pair of regions extending laterally in the width direction WD from the side edges of the absorber body 56, and each side flap extends in the front-back direction LD from the area overlapping the stretchable lower torso region of the front body section F (the region containing the under-waist elastic members 15 in the illustrated embodiment) to the area overlapping the stretchable lower torso region of the back body section B (the region containing the under-waist elastic members 15 in the illustrated embodiment). Each side flap 60 has a side nonwoven fabric 66 extending from the top face across the side edge down to the under face of the side flap 60, and elongate gathering elastic members 631 to 633 each extending along the front-back direction LD between a top part 61 and an under part 62 of the side nonwoven fabric 66, or the like. The top part 61 and the under part 62 each extends all over the front-back direction LD of the side flap 60. Each side flap 60 has in its front and back end regions side non-stretchable regions 70 each of which is not contracted in the front-back direction LD together with the gathering elastic members 631 to 633, and has a fixed part 67 wherein the top part 61 is fixed to the under part 62. The region of each side flap 60 between the front and back side non-stretchable regions 70 forms a planar gather region 80, to which the gathering elastic members 631 to 633 are fixed and which is contracted but stretchable in the front-back direction LD together with the gathering elastic members 631 to 633. The underpants-type disposable diaper of the present invention is devoid of three-dimensional gathers that stand up on the top side, and is provided with only the planar gathers of the side flaps 60. In each fixed part 67 in the illustrated embodiment, the top part 61 is indirectly fixed to the under part 62 via the liquid-impervious film layer 64, but where no other member, such as the liquid-impervious film layer 64, is interposed, the top part 61 is directly fixed to the under part 62.

The dimensions of the side non-stretchable regions 70 in the front-back direction LD may be the same or different between the front and back end regions of each side flap 60.

Characteristically, the fixed part 67 in each side non-stretchable region 70 is spaced apart from the side edge of the side non-stretchable region 70 toward the center in the width direction WD by a first distance w1, and between the side edge and the fixed part 67 of the side non-stretchable region 70 is a hollow area 70h all over its dimension in the width direction WD. Further, the side non-stretchable region 70 has a free zone 71 not fixed to the outer member 12 and extending from the side edge of the side non-stretchable region 70 toward the center in the width direction WD by a second distance w2, which is larger than half the dimension w3 in the width direction WD of the fixed part 67 and smaller than half the first distance w1. The under part 62 located between the free zone 71 and the fixed part 67 is joined to the stretchable lower torso region of the outer member 12 via a hot melt adhesive HM3 or the like, and is contracted but stretchable in the width direction WD together with the lower torso elastic members, and the maximum elongation in the width direction WD of the stretchable lower torso region is 200 to 320%. In the hollow area 70h, the members adjacent to each other in the thickness direction are not joined, and in the illustrated embodiment, the top part 61 is not fixed to the liquid-impervious film layer 64 nor to the under part 62 adjacent underside of the top part 61. However, suitable modification may be made, for example, to fix the liquid-impervious film layer 64 to the top part 61 and not to join the under part 62 to the liquid-impervious film 11.

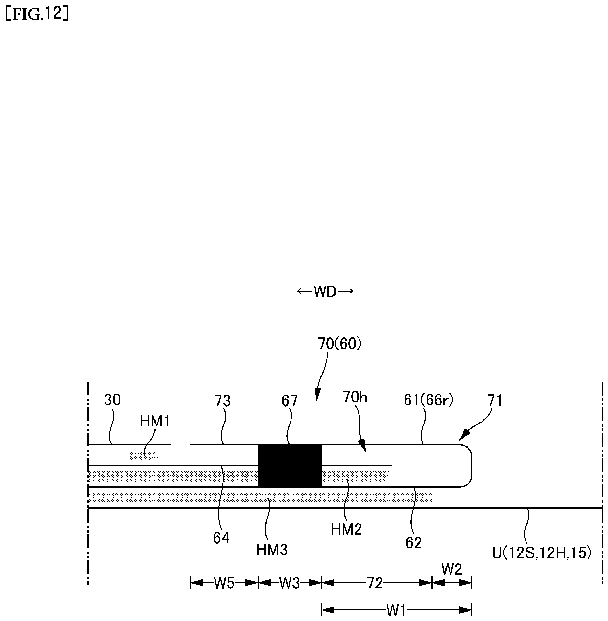

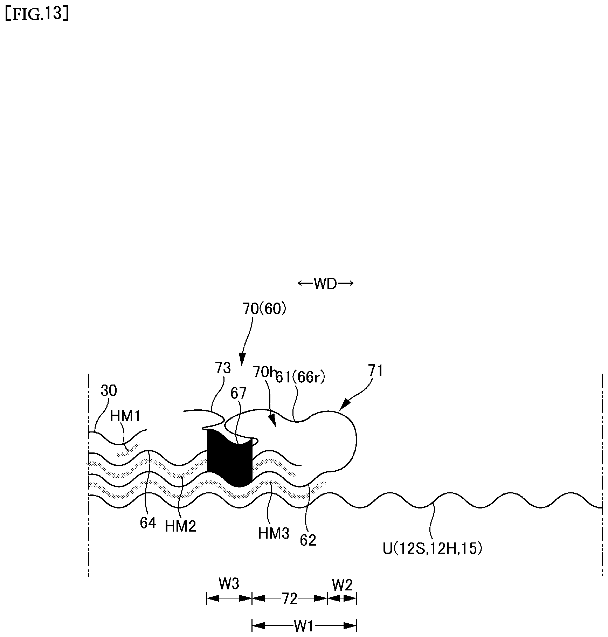

With such a structure, supposing that the maximum elongation in the width direction WD of each stretchable lower torso region in the spread state as shown in Fig. 12 is Emax, when each stretchable lower torso region is in a state of about 0.5 to 0.7 Emax (equivalent to the worn state), in each of the side non-stretchable regions 70, as shown in Fig. 13, the under part 62 of the zone between the free zone 71 and the fixed part 67 is contracted in the width direction WD together with the stretchable lower torso region, which causes the under part 62 of the free zone 71 to stand up, while the top part 61 of the free zone 71 rises and billows. The billowing is flattened and extended when the diaper is worn, and the area of the top part 61 adjacent to the fixed part 67 (non-fixed area of the side nonwoven fabric) hangs over at least the side edge area of the surface of the fixed part 67. As a result, the surface of the hard fixed part 67 in the side non-stretchable region 70 is hard to be brought into contact with the skin, which reduces deterioration of the texture.

The dimensions of various areas, zones, and regions or the like may suitably be decided. The dimension w3 in the width direction WD of the fixed parts 67 is preferably 3 mm or more, particularly 3 to 5 mm. The first distance w1 is preferably 15 to 20 mm. The second distance w2 is preferably one third or less of the first distance w1, and in particular, where 0.3(w1+w2)/2 ≥ w3, the billowing in the top part 61 is larger, which is preferred for readily covering the fixed part 67 widely.

Particularly, the second distance w2 is preferably larger than the dimension in the width direction WD of the fixed part 67 for facilitating billowing of the top part 61.

The dimension in the front-back direction LD of the fixed parts 67 may suitably be decided, and may preferably be about 0.8 to 1.0 time the dimension in the front-back direction LD of the side non-stretchable regions 70. In the illustrated embodiment, the end of each fixed part 67 on the planar gather region 80 side is spaced apart from the planar gather region 80 (e.g., spaced apart by 0.05 to 0.2 times the dimension in the front-back direction LD of the side non-stretchable region 70), while the other end of the fixed part 67 is coincident with an end of the inner body 200. However, this may be vice versa, or both ends in the front-back direction LD of each fixed part 67 may be coincident with the corresponding ends in the front-back direction of the side non-stretchable region 70, or may be spaced apart from the corresponding ends in the front-back direction of the side non-stretchable region 70 (e.g., spaced apart by 0.02 to 0.1 time the dimension in the front-back direction LD of the side non-stretchable region 70) .

In each side non-stretchable region 70, an area of the under part 62 included in the fixed part 67 or located closer to the center in the width direction WD than the fixed part 67 may not be fixed to the outer member 12. However, when the area of the under part 62 included in the fixed part 67 is joined to the stretchable lower torso region of the outer member 12 via a hot melt adhesive HM3 or the like, and is contracted but stretchable in the width direction WD together with the lower torso elastic members, the fixed part 67 is contracted in the width direction WD together with the stretchable lower torso region as shown in Fig. 13, so that the coverage of the fixed part 67 with the top part 61 is relatively larger. Thus, the surface of the fixed part 67 is more hardly brought into contact with the skin, which is preferred.

Each fixed part 67 is spaced laterally apart from a side edge of the absorber body 56 by a third distance w4, and the top part 61 may have a terminal zone 73 protruding from the fixed part 76 centrally in the width direction WD by a fourth distance w5, which is shorter than the third distance w4, and not joined to any member adjacent underside thereof. When the fourth distance w5 is larger than half the dimension in the width direction WD of the fixed part 67, and the area of the under part 62 located between the fixed part 67 and a side edge of the absorber body 56 is joined to the stretchable lower torso region of the outer member 12 via a hot melt adhesive HM3 or the like, and is contracted but stretchable in the width direction WD together with the lower torso elastic members, the terminal zone 73 of the top part 61 protruding from the fixed part 67 centrally in the width direction WD rises along contraction creases of the member located underside thereof as shown in Fig. 13, and the risen area of the top part 61 adjacent to the fixed part 67 (non-fixed area of the side nonwoven fabric) hangs over at least the side edge area of the surface of the fixed part 67 closer to the center in the width direction WD. As a result, the surface of the fixed part 67 is more hardly brought into contact with the skin, which is preferred.

The dimensions of various areas, zones, and regions or the like may suitably be decided. The third distance w4 is preferably about 5 to 8 times the dimension w3 in the width direction WD of the fixed part 67. The fourth distance w5 is preferably about 0.3 to 0.5 times the third distance w4.

Each side flap 60 preferably has the planar gather region 80 extending from lateral to the maximum width zone 54 of the absorber body 56 located forward of the crotch section M to lateral to the maximum width zone 54 of the absorber body 56 located backward of the crotch section M. The planar gather region 80 contains the elongate gathering elastic members 631 to 633 each extending in the front-back direction LD, and is contracted but stretchable in the front-back direction LD together with the gathering elastic members 631 to 633.

In order to reduce the impact of the leg movements on the lateral sides of the absorber body 56, the side flaps 60 are preferably sufficiently large in width. The width 60w of each side flap 60 is preferably 0.1 to 0.5 times, more preferably 0.3 to 0.5 times, particularly preferably 0.4 to 0.5 times the maximum width 56X of the absorber body 56. Further, the arrangement of the gathering elastic members 631 to 633 may suitably be decided, and preferably as in the illustrated embodiment, wherein the gathering elastic members 631 to 633 are composed of a first gathering elastic member 631 attached at a first distance d1 laterally from the side edge of the maximum width zones 54 of the absorber body 56, a second gathering elastic member 632 attached at a second distance d2 laterally from the first gathering elastic member 631, and a plurality of third gathering elastic members 633 attached at a third distance d3 laterally from the second gathering elastic member 632 and laterally at third intervals d3 thereafter up to a lateral side of the side flap 60 (that is, no other elastic members are provided), with the second distance d2 being 1.5 to 5 times, more preferably 1.5 to 2.5 times the third distance d3, and the first distance d1 being 0.3 to 0.8 times, more preferably 0.3 to 0.7 times the second distance d2. More specifically, in the case of a diaper for babies and infants, the width of the side flaps 60 is preferably 30 to 50 mm, particularly preferably 40 to 50 mm.

Where the first gathering elastic member 631 has a first fineness and is attached to a side flap 60 at a first stretch rate, the second gathering elastic member 632 has a second fineness and is attached to the side flap 60 at a second stretch rate, and each third gathering elastic member 633 has a third fineness and is attached to the side flap 60 at a third stretch rate, it is preferred that (a1) the first stretch rate is 0.95 to 1.05 times the second stretch rage and the third stretch rate, and the first fineness is 1.2 to 1.5 times the second fineness and the third fineness; (b1) the first fineness is 0.95 to 1.05 times the second fineness and the third fineness, and the first stretch rate is 1.2 to 1.5 times the second stretch rate and the third stretch rate; or (c1) the first fineness is 1.2 to 1.5 times the second fineness and the third fineness, and the first stretch rate is 1.2 to 1.5 times the second stretch rate and the third stretch rate, and more preferred that (a2) the first stretch rate is 0.95 to 1.05 times the second stretch rate and the third stretch rate, and the first fineness is 1.3 to 1.4 times the second fineness and the third fineness; (b2) the first fineness is 0.95 to 1.05 times the second fineness and the third fineness, and the first stretch rate is 1.3 to 1.5 times the second stretch rate and the third stretch rate; or (c2) the first fineness is 1.3 to 1.4 times the second fineness and the third fineness, and the first stretch rate is 1.3 to 1.5 times the second stretch rate and the third stretch rate.

The gathering elastic members 631 to 633 may be elongate elastic members, such as rubber threads or rubber bands. When rubber threads are used, the first to third finenesses may be preferably 400 to 950 dtex, more preferably 470 to 780 dtex. The first to third stretch rates may be preferably 200 to 320%, more preferably 260 to 320%.

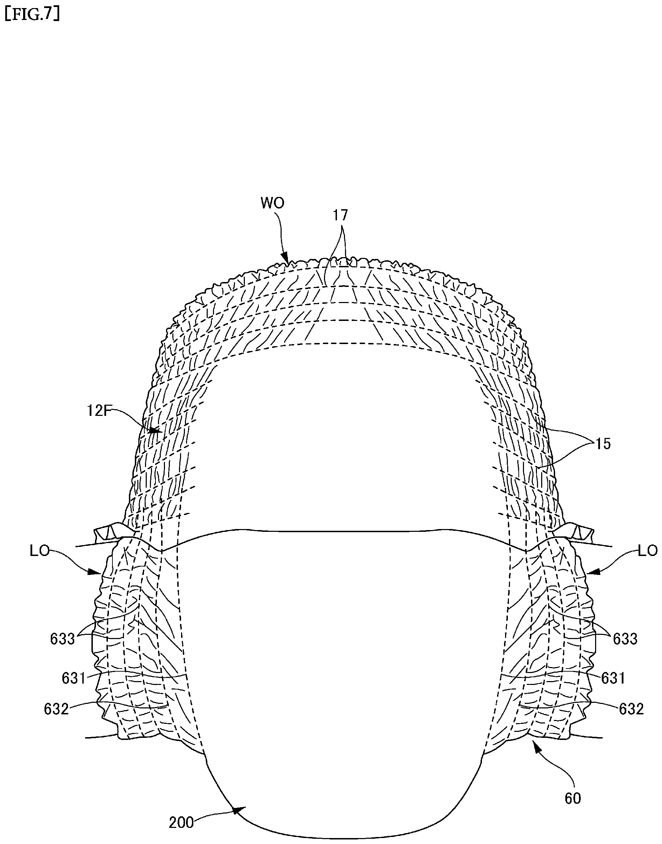

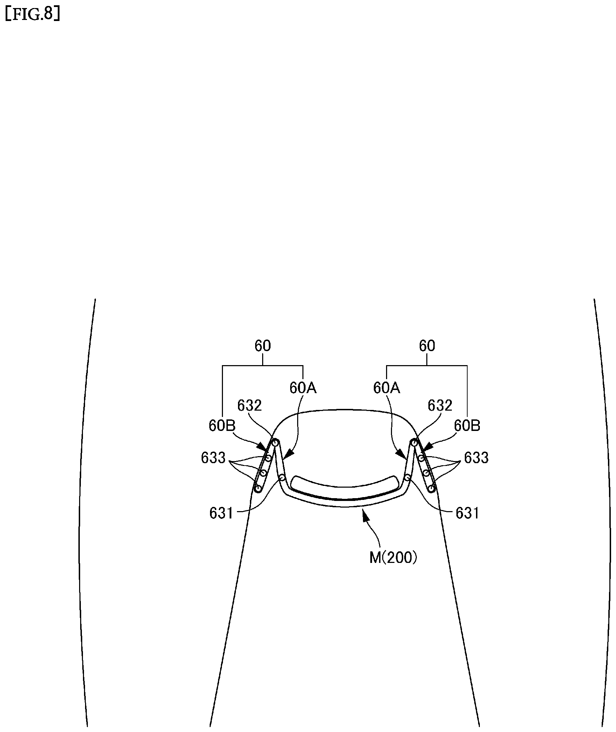

In this way, with sufficiently large width of the side flaps 60 having the planar gathers being ensured, where the first gathering elastic member 631 is disposed near the absorber body 56, the second gathering elastic member 632 is disposed laterally spaced apart therefrom to some extent, and the plurality of third gathering elastic members 633 are disposed at smaller intervals up to the lateral side of the side flap 60, while the force required for stretching the first gathering elastic member 631 disposed near the absorber body 56 is larger than the force required for stretching the second gathering elastic member 632 and the third gathering elastic members 633, the diaper in an appropriately worn state is as shown in Figs. 7 and 8. That is, in the crotch section M, the zone located between the side edge of the absorber body 56 and the second gathering elastic member 632 forms a first zone 60A, which extends obliquely upward toward the groin of the wearer, and the zone between the second gathering elastic member 632 and the side edge of the side flap 60 forms a second zone 60B, which extends obliquely downward along the inner thigh. Here, the first zone 60A is raised and supported more strongly by the first gathering elastic member 631 and is in a hardly displaceable state compared to the second zone 60B, which is apart from the first gathering elastic member 631 beyond a certain distance and is brought into close contact with the inner thigh, supported by the first gathering elastic member 631. Accordingly, even when the second zone 60B moves following the movement of the leg, the zone between the first and second gathering elastic members 631 and 632 deforms to produce a buffering effect, so that the movement of the second zone 60B is hard to be transferred via the first zone 60A to the absorber body 56, to thereby reduce the risk of deterioration of the lateral sides of the absorber body 56.

As in the illustrated embodiment, where the absorber body 56 has the narrowest zone 52 in the crotch section M and enlarging zones 53 extending forward or backward from the narrowest zone 52 toward maximum width zones 54 with increasing width, with too large a distance d4 in the width direction WD between the side edge of the narrowest zone 52 of the absorber body 56 and the first gathering elastic member 631, the area between the side edge of the narrowest zone 52 of the absorber body 56 and the first gathering elastic member 631 may tend to be largely depressed to the underside, the bodily waste may likely be collected on the side flaps 60, or the toes of the wearer, upon putting the diaper on, may likely be caught on the side flaps 60. It is preferred that the distance d4 in the width direction WD between the side edge of the narrowest zone 52 of the absorber body 56 and the first gathering elastic member 631 is 1.1 to 1.5 times, particularly 1.1 to 1.3 times the second distance d2.

The structure of the side flaps 60 may suitably be decided, and the illustrated embodiment is preferred for its simpleness, waterproof property, and easiness of production. That is, each side flap 60 of the illustrated embodiment has a top nonwoven layer constituting the top face of the side flap 60 (the side area of the top sheet 30 and the top part 61 of the side nonwoven fabric 66 in the illustrated embodiment), an under nonwoven layer constituting the under face of the side flap 60 (the under part 62 of the side nonwoven fabric 66 in the illustrated embodiment), the gathering elastic members 631 to 633 each extending in the front-back direction between the top and under nonwoven layers, and the liquid-impervious film layer 64 extending between the top and under nonwoven layers from the base end toward the outer edge. In the illustrated embodiment, each side flap 60 has a no-nonwoven zone 65 in part of its width WD, where the top nonwoven layer is not present and the liquid-impervious film layer 64 is exposed but, for example, the top nonwoven layer may extend almost all over the width WD of the side flap 60 by extending the top part 61 of the side nonwoven fabric 66 up to the lateral side of the top sheet 30. The width of the no-nonwoven zone 65, if provided, may suitably be decided, and preferably 10 mm or less. The liquid-impervious film layer 64 between the top and under nonwoven layers may be omitted where the water shieling property may be ensured only with the top and under nonwoven layers. Where the liquid-impervious film 11 is provided, the liquid-impervious film layer 64 may be fixed or not fixed to one or both of the top nonwoven layer and the under nonwoven layer, as long as the gathering elastic members 631 to 633 are fixed and the hollow area 70h discussed above is formed.

In particular, with the zone 65 where the top nonwoven layer is not present and the liquid-impervious film layer 64 is exposed (including a cut with little width in the top nonwoven layer), which is rendered less stiff, the zone between the first gathering elastic member 631 and the second gathering elastic member 632 is particularly deformable, which is preferred.

The zone between the first gathering elastic member 631 and the second gathering elastic member 632, which produces the buffering effect as mentioned above, is preferably less stiff. As in the illustrated embodiment, where the zone between the first gathering elastic member 631 and the second gathering elastic member 632 is in a three-layered structure of the top nonwoven layer, under nonwoven layer, and the liquid-impervious film layer 64, wherein the top nonwoven layer and the liquid-impervious film layer 64 is bonded with a first hot melt adhesive HM1 and the under nonwoven layer and the liquid-impervious film layer 64 is bonded with a second hot melt adhesive HM2, the top nonwoven layer and the under nonwoven layer each has a bending resistance of preferably 0.2 to 2.2 mN·cm, particularly preferably 0.4 to 0.6 mN.cm in the front-back direction LD and preferably 0.05 to 0.5 mN·cm, particularly preferably 0.07 to 0.1 mN.cm in the width direction WD, as determined by 41.5° cantilever testing method provided in JIS L 1913; 2010. The liquid-impervious film layer 64 has a bending resistance of preferably 0.006 to 0.05 mN·cm, particularly preferably 0.006 to 0.02 mN·cm in the front-back direction LD, and preferably 0.006 to 0.05 mN·cm, particularly preferably 0.006 to 0.02 mN·cm in the width direction WD, as determined by 41.5° cantilever testing method provided in JIS L 1913; 2010. The amount of the first hot melt adhesive HM1 applied is preferably 1 to 10 g/m², particularly 1 to 5 g/m², and the amount of the second hot melt adhesive HM2 applied is preferably 1 to 10 g/m², particularly 1 to 5 g/m².

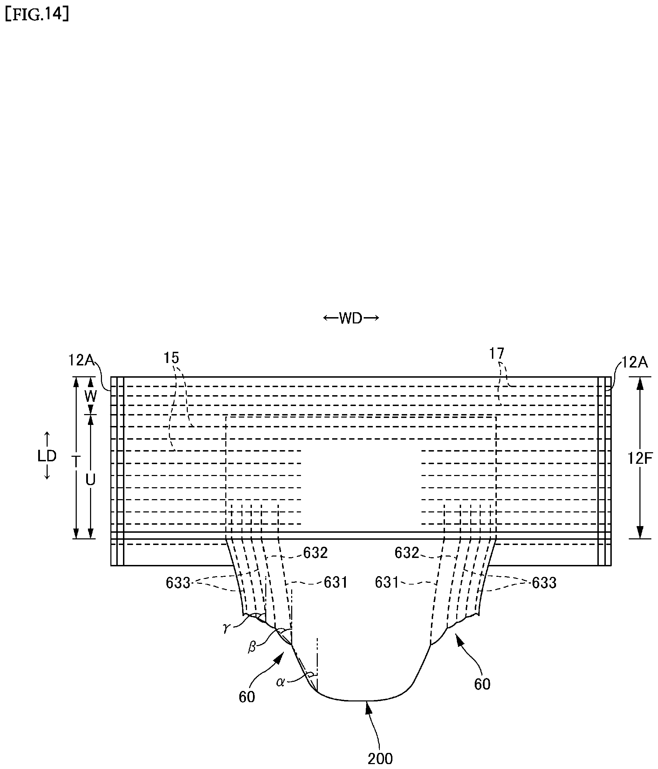

The above-mentioned buffering effect is advantageously greater where, on the contour of the crotch section M seen from the above when the diaper is placed on a horizontal surface with the back body section down, and unfolded from the natural length state into the spread state only in the outer members 12 on the flat surface as shown in Fig. 14, the intersection angle α on the acute angle side formed by a virtual straight line connecting a side edge of the absorber body 56 and the first gathering elastic member 631 with respect to the front-back direction LD is 15 to 40 degrees, more preferably 25 to 35 degrees, the intersection angle γ on the acute angle side formed by a virtual straight line connecting adjacent third gathering elastic members 633 with respect to the front-back direction LD is 50 to 90 degrees, more preferably 55 to 70 degrees, the intersection angle β on the acute angle side formed by a virtual straight line connecting the first gathering elastic member 631 and the second gathering elastic member 632 with respect to the front-back direction LD is larger than α and smaller than γ, more preferably about 1.3 to 1.7 times of α.

In the fixed part 67 in each side non-stretchable region 70, the top part 61 may be fixed to the under part 62 indirectly (in the illustrated embodiment, the liquid-impermeable film layer 64 is interposed between the top part 61 and the under part 62) or directly. The fixed part 67 may be formed by at least one of the hot melt adhesive and the material melt-bonding (such as heat sealing or ultrasonic sealing). In the illustrated embodiment, the fixed part 67 is envisaged to be formed by integrally melt-bonding the top and under parts 61 and 62 of the side nonwoven fabric 66 and the liquid-impervious film layer 64 interposed therebetween. Each side non-stretchable region 70 may be provided partially coincident with the stretchable lower torso region of the outer members 12, and may preferably be provided entirely coincident with the stretchable lower torso region of the outer member 12. Each planar gather region 80 (the region of the side flap 60 stretchable with the gathering elastic members 631 to 633) takes up the entire region between the front and back side non-stretchable regions 70.

The gathering elastic members 631 to 633, as long as positioned between the top and under nonwoven layers (thus not provided in the no-nonwoven zone 65), may be positioned on the top side of the liquid-impervious film layer 64 contained in the side flap 60 as shown in Fig. 3, or on the back side thereof, though not shown. As long as the contracting force of the gathering elastic members 631 to 633 does not act on the side non-stretchable regions 70, the ends of the gathering elastic members 631 to 533 contracted to the natural length may not be fixed to the neighboring parts.

The liquid-impervious film layer 64 may be arranged suitably in the side flaps 60 and, when the waterproof property is emphasized, the liquid-impervious film layer 64 preferably extends all over the width of the side flaps or, for the purpose of ensuring flexibility of the side edges of the side flaps, the liquid-impervious film layer 64 is also preferably not present in the lateral sides of the side flaps (e.g., from between the lateral-most third gathering elastic member and the third gathering elastic member adjacent thereto, i.e., the second from the lateral-most, to the side edge of each side flap).

The top nonwoven layer and the under nonwoven layer may be made of nonwoven fabric other than those for the top sheet 30 or the covering nonwoven layer 13, or may preferably be made of the same nonwoven fabric used for the top sheet 30 or the covering nonwoven layer 13. That is, in the illustrated embodiment, the top sheet 30 is made of nonwoven fabric, which also extends on its lateral sides in the width direction WD beyond the side edges of the absorber body 56, whereas on the underside of the absorber body 56 is disposed the side nonwoven fabric 66, which is also the covering nonwoven layer 13, and extends laterally on its lateral sides in the width direction WD beyond the side edges of the absorber body 56, and is folded back on the lateral sides, with the edge of each folded part 66r being spaced apart from the edge of the top sheet 30. Further, the liquid-impervious film 64 is disposed at least from between the folded nonwoven fabric 66 in the folded part 66r to between the top sheet 30 and the nonwoven fabric 66 on each lateral side. As a result, the part of the side nonwoven fabric 66 other than the folded parts 66r constitutes the under nonwoven layer including the under part 62, the folded parts 66r of the side nonwoven fabric 66 and the parts of the top sheet 30 extending laterally beyond the absorber body 56 constitute the top nonwoven layer, and the zone where each folded part 66r of the side nonwoven fabric 66 is spaced apart from the top sheet 30 constitutes the no-nonwoven zone 65. In this way, by forming the top nonwoven layer on the absorber body 56 side of each no-nonwoven zone 65 with the top sheet 30, while forming the remaining part of the top nonwoven layer with the folded parts 66r of the side nonwoven fabric 66, the no-nonwoven zones 65 may be provided without cutting of the material to simplify the structure and make the production easier.

In this case, where the liquid-impervious film layer 64 contained in the side flaps 60 may preferably extend from one of the side flaps 60 across the underside of the absorber body 56 to the other of the side flaps 60 as shown in Figs. 3 and 4, for integrally ensuring not only the water shielding property of the side flaps 60, but also the water shielding property on the underside of the absorber body 56. However, the liquid-impervious film layer 64 contained in the side flaps 60 may separately be provided from the liquid-impervious film layer 11 covering the underside of the absorber body 56. In the latter case, the material of the liquid-impervious film layer 64 contained in the side flaps 60 may be the same as or different (e.g., with a lower stiffness) from that of the liquid-impervious film layer 11 covering the underside of the absorber body 56.

For bonding adjacent layers in the thickness direction of the side flaps 60, or fixing the gathering elastic members 631 to 633 interposed therebetween, at least one of the fixing means including a hot melt adhesive applied in a various manner and material melt-bonding, such as heat sealing or ultrasonic sealing may be employed. If the layers adjacent to the gathering elastic members 631 to 633 on their top side and underside are bonded all over, the flexibility of the layers are impaired, so that it is preferred not to bond or to bond weakly the layers in the areas other than the bonding areas of the gathering elastic members 631 to 633. In the illustrated embodiment, by applying a hot melt adhesive only to the circumferential surfaces of the gathering elastic members 631 to 633 using application means, such as comb guns or surewrap nozzles, and holding the gathering elastic members 631 to 633 between the layers adjacent thereto on their top side and underside, not only the fixing of the gathering elastic members 631 to 633 to the sheet layers adjacent thereto on their top side and underside, but also the fixing between the layers adjacent to the gathering elastic members on their top side and underside, may be achieved by means of only the hot melt adhesive applied on the circumferential surfaces of the gathering elastic members 631 to 633. Where nonwoven fabric or sheet is separated from the gathering elastic members 631 to 633 as in the edge areas of the top sheet 30 in the illustrated embodiment, a hot melt adhesive (first hot melt adhesive HM1 in the illustrated embodiment) may separately be applied for fixing the same.

The side nonwoven fabric 66 may preferably be made of flexible nonwoven fabric having good uniformity and concealability, such as spunbonded nonwoven fabric (SS, SSS, or the like), SMS nonwoven fabric (SMS, SSMMS, or the like), or melt-blown nonwoven fabric, subjected to water-repellent treatment with, e.g., silicone, as required, and having a fiber basis weight of about 10 to 30 g/m². As may be seen from the fact that part of the top nonwoven layer centrally to the no-nonwoven zone 65 is formed of the top sheet 30 in the embodiment illustrated in Figs. 3 and 4, the top nonwoven layer and the under nonwoven layer may partially be made of different materials, or the top nonwoven layer and the under nonwoven layer may be made of different materials.

### <Compressed Area>

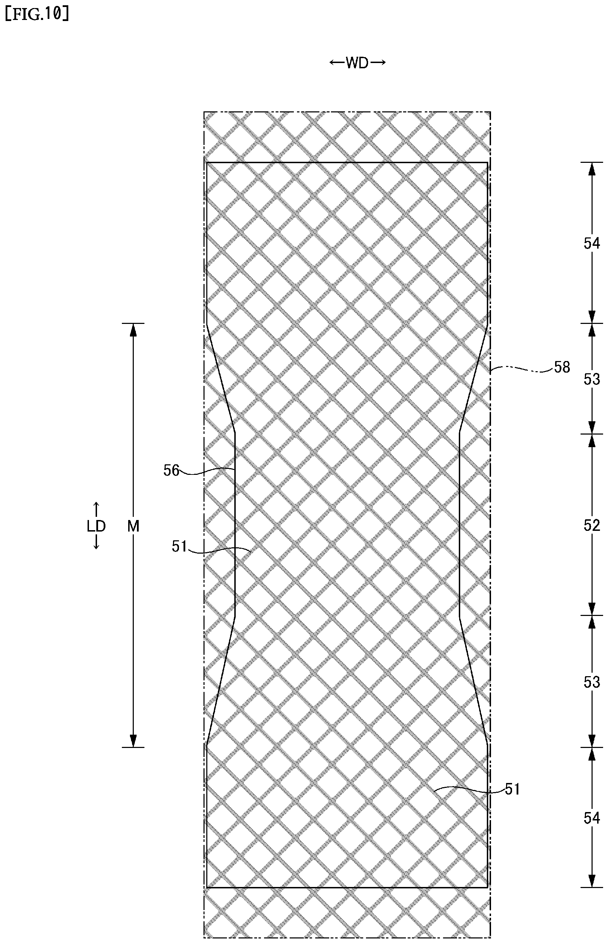

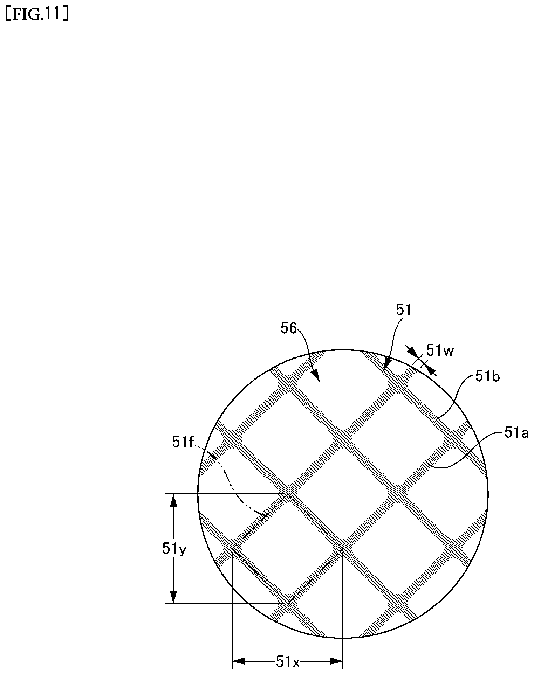

In order to improve the shape-retaining property, the absorber body 56 may be provided with compressed areas wherein the absorber body 56 is compressed in its thickness direction. The compressed areas of the absorber body 56 may be formed by embossing only the absorber body 56 under or without heating or the like, or the compressed areas 51 may be formed on the absorber body 56 together with the other layered members by embossing the absorber body 56 together with the other members layered thereon, like the absorbent element 50 (i.e., together with the packing sheet 58) or the inner member 200. The compressed areas 51 may preferably be provided in a grid-like continuous pattern as shown in Figs. 10 and 11, or in a scattered and spaced dotted pattern, though not shown.

The dimensions and the number of the compressed areas 51 may suitably be decided and, when each compressed area 51 has a bottom face and side faces, the depth 51h of each compressed area 51 may preferably be 1 to 5 mm, and the percentage of the bottom faces of the compressed areas 51 with respect to the surface of the absorbent element 50 may preferably be 5 to 25%. When the compressed areas 51 are arranged in a scattered and spaced pattern, the area of the bottom face of each compressed area 51 may suitably be decided and may be 5 to 15 mm². The shape of the bottom face of the compressed areas may be one or more shapes selected from suitable shapes, such as circle, ellipse, polygon, rectangle, and rounded rectangle (defined by two parallel lines of equal length and two semicircles). The scattered and spaced pattern of the compressed areas 51 may include not only regular patterns, such as a grid-like pattern as well as a scattered pattern or a matrix pattern, but also an irregular pattern.

In particular, with the compressed areas 51 arranged in a grid-like continuous pattern, the pulp fibers and high-absorbent polymer particles constituting the absorber body 56 may be confined in the unit frames 51f defined by the compressed areas 51 (see Fig. 11), so that distortion or cracking of the absorber body 56 may be avoided.

When the compressed areas 51 are to be provided in a grid-like pattern, the width 51w of the compressed areas 51 in the form of grooves (width of the bottom face of the compressed areas formed on the absorbent element 50) may suitably be decided and may preferably be about 1 to 3 mm.

The thickness 51t of the compressed areas 51 may suitably be decided, and may usually be preferably 15 to 35% the maximum of the thickness 50t of the absorbent element 50.

The shape of each unit frame is not particularly limited, and may be approximate square as shown in Figures, or other polygon, such as approximate rhomboid (exclusive of square), approximate rectangle, approximate square, or approximate triangle. Further, unit frames 51f of different shapes may be included as long as the compressed areas 51 are arranged in a grid-like pattern.

One preferred grid-like pattern of the compressed areas 51 may be a lattice pattern composed of, as in the embodiment shown in Figs. 10 and 11, first areas 51a each extending obliquely at 40 to 50° clockwise with respect to the front-back direction LD in plan view, and second areas 51b each extending obliquely at 40 to 50° counterclockwise with respect to the front-back direction LD in plan view. In this case, the shape of each unit frame 51f is approximate rhombic.

The size of each unit frame 51f may suitably be decided. If too large, the unit frames may have poor rigidity improving effect, and if too small, the unit frames may be too rigid. Thus, usually, the dimension 51x in the width direction WD of each unit frame 51f may preferably be about 15 to 20 mm, and the dimension 51y in the front-back direction LD of each unit frame 51f may preferably be about 15 to 20 mm.

The compressed areas 51 may be formed all over or only in part of the absorbent element 50.

### <Explanation of Terms in the Specification>

The following terms appearing in the present specification shall have the following means unless otherwise specified herein.
- The "front-back direction" refers to the direction shown by the reference sign LD (longitudinal direction) in the figures, whereas the "width direction" refers to the direction shown by the reference sign WD (transverse direction) in the figures, and the front-back direction and the width direction are orthogonal to each other.
- The "top side" refers to the side, when the article is worn, closer to the skin of the wearer, whereas the "underside" refers to the side, when the article is worn, away from the skin of the wearer.
- The "top face" refers to the face, when the article is worn, closer to the skin of the wearer, whereas the "under face" refers to the face, when the article is worn, away from the skin of the wearer.
- The "stretch rate" refers to a value with respect to the natural length being 100%. For example, a 200% stretch rate is synonymous with stretch in two folds.
- The "gel strength" is determined as follows. To 49.0 g of artificial urine (a mixture of 2 wt% urea, 0.8 wt% sodium chloride, 0.03 wt% calcium chloride dihydrate, 0.08 wt% magnesium sulfate heptahydrate, and 97.09 wt% ion-exchanged water), 1.0 g of superabsorbent polymer is added and stirred with a stirrer. The resulting gel is left in a chamber with constant temperature and humidity at 40 °C at 60% RH for 3 hours, and then the temperature is returned to the ordinary temperature. The gel strength is measured in a curd meter (Curdmeter-MAX ME-500 manufactured by I. techno Engineering).
- The "basis weight" is determined as follows. A specimen or test piece is preliminarily dried, left in a laboratory or in apparatus under the standard conditions (23 ± 1 °C temperature and 50 ± 2% relative humidity in the testing location) until constant mass is attained. The preliminary drying refers to attaining constant mass from a specimen or test piece in the environment at a temperature of 100 °C. No preliminary drying may be performed on fibers with an official regain of 0.0%. From the test piece of the constant mass, a specimen of 100 mm × 100 mm size is cut out using a sampling template (100 mm × 100 mm). The weight of the specimen is measured and multiplied by 100 times to calculate the weight per 1 m², which is taken as the basis weight.
- The "thickness" of a thick member, such as the absorber body 56, the absorbent element 50, or the bottom face of the compressed areas 51, is measured using a thickness meter manufactured by OZAKI MFG. CO., LTD. (PEACOCK, dial thickness gauge Model H (measurement range of 0 to 10 mm, circular terminal with measurement area of 10 mm in diameter, measuring force of about 1.7 N, pressure of about 21.7 KPa)), with the specimen and the thickness meter kept horizontally.
- The "thickness" of a thin sheet, such as nonwoven fabric, is automatically measured using an automatic thickness meter (KES-G5 handy compression tester program) under a load of 0.098 N/cm² with the compression area of 2 cm².
- The water absorption is determined in accordance with JIS K7223 - 1996 "Testing method for water absorption capacity of super absorbent polymers".
- The water absorption rate is defined as the "time spent until the end point is reached" in carrying out JIS K7224 - 1996 "Testing method for water absorption rate of super absorbent polymers" using 2 g of superabsorbent polymer and 50 g of saline.
- The "spread state" refers to the state in which an article is spread flatly without contraction (including any contraction, such as contraction by means of elastic members) or slack.
- The "maximum elongation" refers to the maximum value of elongation of a stretchable region in the stretching-contracting direction (i.e., elongation at elastic limit, equivalent to the elongation in the spread state), and expressed in percentage with respect to the natural length being 100%.
- The size of each part refers to the size not in the natural length state but in the spread state, unless otherwise specified.
- A test or measurement shall be, in the absence of description about environmental conditions, performed in a laboratory or in apparatus under the standard conditions (23 ± 1 °C temperature and 50 ± 2% relative humidity in the testing location).

### INDUSTRIAL APPLICABILITY

The present invention is applicable to underpants-type disposable wearing articles, such as underpants-type disposable diapers in the embodiments discussed above.

### DESCRIPTION OF REFERENCE SIGNS

11: liquid-impervious film
12: outer member
12A: side seal
12B: back outer member
12F: front outer member
12H: inner sheet layer
12S: outer sheet layer
13: covering nonwoven layer
14: buttocks-covering zone
15: under-waist elastic member
16: buttocks-covering elastic member
17: waist elastic member
200: inner member
201: inner member joint area
30: top sheet
50: absorbent element
51: compressed area
52: narrowest zone
53: enlarging zone
54: maximum width zone
56: absorber body
56C: central region
56S: side region
58: packing sheet
60: side flap
60A: first zone
60B: second zone
61: top part
62: under part
631-633: gathering elastic members
631: first gathering elastic member
632: second gathering elastic member
633: third gathering elastic member
64: liquid-impervious film layer
65: no-nonwoven zone
66: side nonwoven fabric
66r: folded par
67: fixed part
70: side non-stretchable region
701h: hollow area
71: free zone
73: terminal zone
80: planar gather region
B: back body section
F: front body section
HM1: first hot melt adhesive
HM2: second hot melt adhesive
HM3: hot melt adhesive
L: intermediate region
LD: front-back direction
M: crotch section
T: lower torso region
U: under-waist zone
W: waist zone
WD: width direction
WO: waist opening
d1: first distance
d2: second distance
d3: third distance
w1: first distance
w2: second distance
w4: third distance
w5: fourth distance

## Claims

1. An underpants-type disposable wearing article, comprising:
an outer member comprising a lower torso section of a front body section, a lower torso section of a back body section, and side seals formed by joining the two sections along their lateral sides opposed in a width direction,
an inner member joined to a middle of the outer member in the width direction to extend from the lower torso section of the front body section, via a crotch section, to the lower torso regio of the back body section,
a waist opening defined by a front edge of the front body section and a back edge of the back body section, and
a pair of leg openings each formed on either lateral side of the inner member,
wherein the inner member comprises an absorber body extending from forward of the crotch section to backward of the crotch section,
wherein the lower torso section of the front body section and the lower torso section of the back body section each comprises stretchable lower torso regions provided at least on lateral sides opposed in the width direction,
wherein the stretchable lower torso regions each contains lower torso elastic members, and is contracted but stretchable in the width direction WD together with the lower torso elastic members,
wherein the inner member has on its opposed lateral sides a pair of side flaps extending laterally in the width direction from the side edges of the absorber body, each side flap extending in the front-back direction from an area overlapping the stretchable lower torso region of the front body section to an area overlapping the stretchable lower torso region of the back body section,
wherein each side flap has a side nonwoven fabric extending from a top face across a side edge down to an under face of the side flap, and elongate gathering elastic members each extending along the front-back direction between a top part and an under part of the side nonwoven fabric,
wherein the top part and the under part each extends all over the front-back direction of the side flap,
wherein each side flap has in its front and back end regions side non-stretchable regions each of which is not contracted in the front-back direction together with the gathering elastic members, and has a fixed part wherein the top part is directly or indirectly fixed to the under part,
wherein a region of each side flap between the front and back side non-stretchable regions forms a planar gather region, to which the gathering elastic members are fixed and which is contracted but stretchable in the front-back direction together with the gathering elastic members, and
wherein the article is devoid of three-dimensional gathers that stand up on a top side,
wherein the fixed part in each side non-stretchable region is spaced apart from a side edge of the side non-stretchable region toward a center in the width direction by a first distance,
wherein between the side edge and the fixed part of the side non-stretchable region is a hollow area all over its dimension in the width direction,
wherein the side non-stretchable region has a free zone not fixed to the outer member and extending from the side edge of the side non-stretchable region toward the center in the width direction by a second distance,
wherein the second distance is larger than half a dimension in the width direction of the fixed part and smaller than half the first distance,
wherein the under part located between the free zone and the fixed part is joined to the stretchable lower torso region of the outer member, and is contracted but stretchable in the width direction together with the lower torso elastic members, and
wherein a maximum elongation in the width direction of each stretchable lower torso region is 200 to 320%.

2. The underpants-type disposable wearing article according to claim 1,
wherein the first distance is 15 to 20 mm,
wherein the second distance is one third or less of the first distance, and
wherein a dimension in the width direction of the fixed part is 3 mm or more.

3. The underpants-type disposable wearing article according to claim 1 or 2,
wherein, where the first distance is w1, the second distance is w2, and the dimension in the width direction of the fixed part is w3, a formula 0.3 (w1 + w2) / 2 ≥ w3 is satisfied.

4. The underpants-type disposable wearing article according to claim 1 or 2,
wherein the second distance is larger than the dimension in the width direction of the fixed part.

5. The underpants-type disposable wearing article according to claim 1 or 2,
wherein an area of the under part included in the fixed part is joined to the stretchable lower torso region of the outer member, and is contracted but stretchable in the width direction together with the lower torso elastic members.

6. The underpants-type disposable wearing article according to claim 1 or 2,
wherein the fixed part is spaced laterally apart from a side edge of the absorber body by a third distance,
wherein the top part has a terminal zone protruding from the fixed part centrally in the width direction by a fourth distance, and not joined to any member adjacent underside thereof,
wherein the fourth distance is larger than half a dimension in the width direction of the fixed part, and
wherein an area of the under part located between the fixed part and a side edge of the absorber body is joined to the stretchable lower torso region of the outer member, and is contracted but stretchable in the width direction together with the lower torso elastic members.
